(19) Europäisches Patentamt
European Patent Office
Office européen des brevets

(11) Veröffentlichungsnummer : **0 016 391**
**B1**

(12) # EUROPÄISCHE PATENTSCHRIFT

(45) Veröffentlichungstag der Patentschrift : **30.03.83**

(51) Int. Cl.³ : **C 07 D 253/08**, A 61 K 31/53

(21) Anmeldenummer : **80101201.4**

(22) Anmeldetag : **10.03.80**

(54) **3-Sulfonyl-benzo-1,2,4-triazine und -benzo-1,2,4-triazin-1-oxide, Verfahren zu ihrer Herstellung und diese enthaltende Arzneimittel sowie antimikrobielle Mittel für den Materialschutz.**

(30) Priorität : **21.03.79 DE 2910974**

(43) Veröffentlichungstag der Anmeldung : **01.10.80 Patentblatt 80/20**

(45) Bekanntmachung des Hinweises auf die Patenterteilung : **30.03.83 Patentblatt 83/13**

(84) Benannte Vertragsstaaten : **AT BE CH DE FR GB IT NL SE**

(56) Entgegenhaltungen :
**DD C 83 869**
**DE A 2 538 179**
**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Sasse, Klaus, Dr.**
**Pützweg 13**
**D-5060 Bergisch-Gladbach 2 (DE)**
Erfinder : **Haller, Ingo, Dr.**
**Viktoriastrasse 99**
**D-5600 Wuppertal 1 (DE)**
Erfinder : **Plempel, Manfred, Dr.**
**Pahlkestrasse 5**
**D-5600 Wuppertal 1 (DE)**
Erfinder : **Zeiler, Hans-Joachim, Dr.**
**Windrather Strasse 188**
**D-5620 Velbert 15 (DE)**
Erfinder : **Metzger, Karl Georg, Dr.**
**Pahlkestrasse 15**
**D-5600 Wuppertal 1 (DE)**

**EP 0 016 391 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

3-Sulfonyl-benzo-1,2,4-triazine und -benzo-1,2,4-triazin-1-oxide, Verfahren zu ihrer Herstellung und diese enthaltende Arzneimittel sowie antimikrobielle Mittel für den Materialschutz

Die vorliegende Erfindung betrifft neue 3-Sulfonyl-1,2,4-benzotriazine und -benzotriazin-1-oxide, Verfahren zu ihrer Herstellung sowie diese enthaltende Arzneimittel und antimikrobielle Mittel für den Materialschutz.

Es ist bereits bekannt geworden, daß Benzo-1,2,4-triazin-1-oxide, die in 3-Stellung Halogen, eine Amino-, Hydrazino-, Alkoxy- oder Alkylmercapto-Gruppe besitzen, herbizide, akarizide und fungizide Eigenschaften aufweisen [vgl. hierzu DD-C-83 869]. Weiterhin ist bekannt, daß auch 3-Alkoxy-benzo-1,2,4-triazine fungizide und bakterizide Eigenschaften besitzen [vgl. hierzu DE-A-2 538 179]. Verbindungsklassen dieser Art sind jedoch wenig oder gar nicht wirksam gegen Mikroorganismen, die mykotische Krankheiten bei Mensch und Tier auslösen, sowie solche, die zum Befall oder zur Zerstörung organischer Materialien führen. Eine sporozide Wirkung der bekannten Verbindungen wurde bisher nicht beschrieben.

Es wurde gefunden, daß die neuen 3-Sulfonyl-benzo-1,2,4-triazine und -benzotriazin-1-oxide der Formel (I)

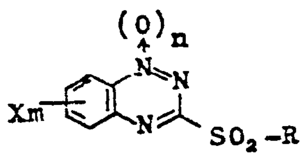

(I)

starke antimykotische Eigenschaften aufweisen. In Formel (I) bedeuten :

X Chlor, Brom und Trifluormethyl,

R $C_1$-$C_4$-Alkyl, einen gegebenenfalls durch Halogen, Nitro oder Methyl substituierten Benzylrest oder einen gegebenenfalls durch Halogen, Nitro, Methyl oder Alkoxy mit 1 bis 4 C-Atomen substituierten Phenylrest,

m 1 oder 2 und

n 0 oder 1.

Weiterhin wurde gefunden, daß man Verbindungen der Formel (I) erhält, wenn man 3-Sulfenyl-benzo-1,2.4-triazine bzw. deren 1-Oxide der Formel (II)

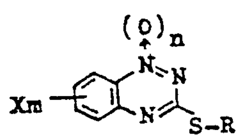

(II)

in der X, R, m und n die oben genannte Bedeutung besitzen, der Oxidation unterwirft.

3-Sulfonyl-benzo-1,2,4-triazine (Formel (I) ; n = 0) sind weiterhin dadurch herstellbar, daß man 3-Sulfonyl-benzo-1,2,4-triazin-1-oxide (Formel (I) ; n = 1) der Reduktion unterwirft.

Ueberraschenderweise zeigen die erfindungsgemäßen 3-Sulfonyl-benzo-1,2,4-triazin(-1-oxid)e eine erheblich höhere antimikrobielle Wirkung als die bekannten Benzotriazine mit anderen Substituenten in 3-Stellung. Außerdem zeigen die erfindungsgemäßen Wirkstoffe überraschenderweise eine zusätzliche sporozide Wirkung auf Pilzsporen. Die erfindungsgemäßen Stoffe stellen somit eine Bereicherung der Pharmazie und des Materialschutzes dar.

Die Herstellungsverfahren laufen formelmäßig nach folgendem Schema ab :

Beispielsweise wird 3-Methylmercapto-7-chlor-benzo-1,2,4-triazin-1-oxid bzw. 3-Phenylmercapto-7-brom-1,2,4-benzotriazin mit Oxidationsmitteln umgesetzt :

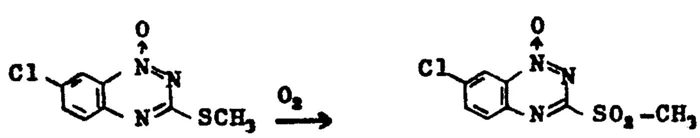


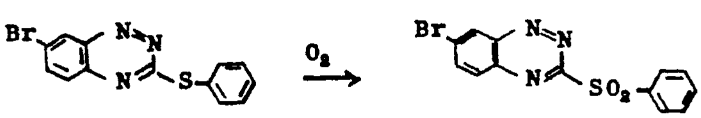


Die in 1-Stellung sauerstoff-freien Verbindungen werden auch durch Reduktion der 1-Oxide gewonnen, z. B. des 3-Methylsulfonyl-7-chlor-benzo-1,2,4-triazin-1-oxids mit katalytisch erregtem Wasserstoff :

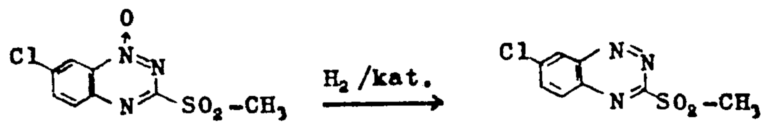


Die als Ausgangsstoffe verwendeten 3-Sulfenyl-benzo-1,2,4-triazin-(1-oxid)e sind durch die allgemeine Formel (II) definiert. In der Formel (II) steht

X für Chlor, Brom und die Trifluormethylgruppe,

R für einen Alkylrest mit 1 bis 4 Kohlenstoffatomen, für den gegebenenfalls durch Halogen, Nitro oder Methyl substituierten Benzylrest oder für den gegebenenfalls durch Halogen, Nitro, Methyl oder Alkoxy mit 1 bis 4 Kohlenstoffatomen substituierten Phenylrest,

m für 1 oder 2 und

n für 0 oder 1.

Die Ausgangsstoffe der Formel (II) sind bisher ebenfalls noch nicht bekannt. Diejenigen mit m = 1 (Formel IIa) werden bevorzugt dadurch hergestellt, daß 3-Chlor-benzo-1,2,4-triazin-1-oxide der Formel (III) mit Mercaptanen der Formel (IV) in Gegenwart säurebindender Mittel umgesetzt werden :

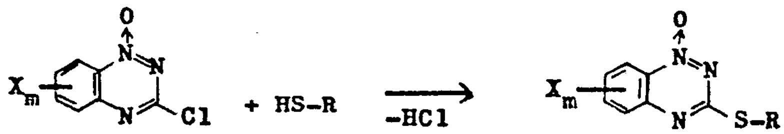


(III) (IV) (IIa)

In diesen Formeln haben X, R und m die gleiche Bedeutung wie oben angegeben.

Die Verbindungen der Formel (III) sind bekannt [vgl. hierzu J. Org. Chem. *24,* 813 (1959) und DOS 2 740 887].

Als Beispiele seien genannt :

3-Chlor-benzo-1,2,4-triazin-1-oxid
3,6-Dichlor-benzo-1,2,4-triazin-1-oxid
3,7-Dichlor-benzo-1,2,4-triazin-1-oxid
3,5,7-Trichlor-benzo-1,2,4-triazin-1-oxid
3-Chlor-7-brom-benzo-1,2,4-triazin-1-oxid
3-Chlor-7-methyl-benzo-1,2,4-triazin-1-oxid
3-Chlor-7-trifluormethyl-benzo-1,2,4-triazin-1-oxid.

Die Verbindungen der Formel (IV) sind ebenfalls bekannt. Vorzugsweise werden der Definition von R entsprechende Alkyl- und Benzylmercaptane bzw. Thiophenole eingesetzt. In der Regel läßt man die Reaktionspartner (III) und (IV) in äquimolaren Verhältnis aufeinander einwirken oder mit einem leichten Ueberschuß von (IV) bis zu 0,25 Mol. Bevorzugt arbeitet man in organischen Lösungsmitteln wie Ketonen, Aethern, auch cyclischen wie Dioxan, Tetrahydrofuran, in Kohlenwasserstoffen, Dimethylformamid und Dimethylsulfoxid oder deren Gemischen mit Wasser. Als säurebindende Mittel verwendet man vorzugsweise Alkali- und Erdalkali-oxide, -hydroxide, -carbonate oder -alkoholate. Die Reaktionstemperaturen betragen normalerweise 0-100 °C.

Die noch nicht bekannten Ausgangsstoffe der Formel (II) mit m = 0 (Formel IIb) werden vorzugsweise durch Reduktion der oben beschriebenen Verbindungen der Formel (IIa) gewonnen, wobei X, R und m die oben angegebene Bedeutung besitzen.

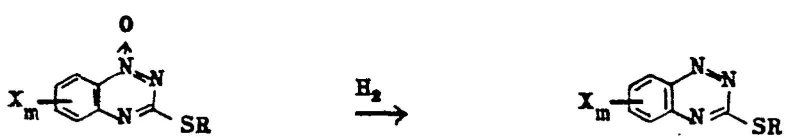


(IIa) (IIb)

Als Reduktionsmittel sind geeignet : Zinkstaub im schwach sauren Medium, Eisen-II-salze, Dithionit oder katalytisch erregter Wasserstoff. Im letzteren Falle werden die bekannten Hydrierkatalysatoren auf Basis Nickel, Cobalt, Palladium, Platin oder Rhodium verwendet. Vorzugsweise verwendet man Raney-Nickel in Mengen von 0,01 bis 5 Mol-%. Die Hydrierung wird in der Regel in geschlossenen Gefäßen bei einem Wasserstoffdruck von 0-25 atm. durchgeführt. Die Reaktionstemperaturen betragen wie auch bei den anderen Reduktionsverfahren 0-100°, vorzugsweise 20-60 °C.

Als Lösungsmittel dienen vorzugsweise Alkohole oder cyclische Aether wie Dioxan und Tetrahydrofuran.

Das Verfahren zur Herstellung der Verbindungen der Formel (I) besteht darin, daß man die Verbindungen der Formel II (IIa bzw. IIb) der Oxidation unterwirft. Als Oxydationsverfahren kommen infrage :

a) Chlor in Anwesenheit von Wasser.

Als Lösungsvermittler werden vorzugsweise niedere aliphatische Carbonsäuren wie Ameisen-, Essig- oder Propionsäure oder Alkohole wie Methanol oder Glykolmonomethyläther verwendet. Es werden mindestens zwei Mol $Cl_2$ für die quantitative Umsetzung benötigt. In der Regel arbeitet man mit einem leichten Ueberschuß bis zu einem weiteren Mol. Die Reaktionstemperatur beträgt 0-20°, vorzugsweise 0-5 °C.

b) Wasserstoffperoxid in schwach saurer Lösung.

Als Lösungsmittel bevorzugt man Essigsäure oder deren mit Wasser verdünnte Lösungen. Für einen quantitativen Umsatz werden 2 Mol $H_2O_2$ benötigt. In der Regel verwendet man einen leichten Überschuß von bis zu 0,5 Mol. Reaktionstemperatur 20-100 °C, vorzugsweise 40-70 °C.

c) Alkali-permanganat

Als Lösungsmittel bevorzugt man Essigsäure oder deren mit Wasser verdünnte Lösungen. Für einen quantitativen Umsatz werden z. B. 1,34 Mol $KMnO_4$ benötigt. In der Regel verwendet man auch hier einen Überschuß bis zu weiteren 0,25 Mol. Die Reaktionstemperaturen betragen 0-100 °C, vorzugsweise 10-50 °C. Vor der Aufarbeitung der Reaktionsansätze empfiehlt es sich, unverbrauchtes Permanganat und entstandenes Mangan-IV-oxid durch Zugabe von Salzen der Schwefligen Säure zu $Mn^{+2}$ zu reduzieren.

Das Verfahren zur Herstellung von Verbindungen der Formel (I), in der n = 0 bedeutet, aus solchen Verbindungen der Formel (I), in der n = 1 bedeutet, besteht darin, daß man eine Reduktionsstufe anschließt, die, wie oben für die Zwischenprodukte der Formel (IIb) beschrieben, durchgeführt wird, d. h. die Reduktion wird unter den dort angegebenen Bedingungen mit Zinkstaub im schwach sauren Medium, mit Eisen-II-salzen, Dithionit oder (bevorzugt) mit katalytisch erregtem Wasserstoff durchgeführt.

Als neue erfindungsgemäße Stoffe seien im einzelnen genannt :

3-Methylsulfonyl-benzo-1,2,4-triazin
3-Methylsulfonyl-benzo-1,2,4-triazin-1-oxid
3-Methylsulfonyl-7-chlor-benzo-1,2,4-triazin
3-Methylsulfonyl-7-chlor-benzo-1,2,4-triazin-1-oxid
3-Aethylsulfonyl-7-chlor-benzo-1,2,4-triazin-1-oxid
3-Butylsulfonyl-7-chlor-benzo-1,2,4-triazin-1-oxid
3-Benzylsulfonyl-7-chlor-benzo-1,2,4-triazin-1-oxid
3-(4-Chlorbenzylsulfonyl)-7-chlor-benzo-1,2,4-triazin-1-oxid
3-(4-Methyl-benzylsulfonyl)-7-chlor-benzo-1,2,4-triazin-1-oxid
3-(3-Nitro-benzylsulfonyl)-7-chlor-benzo-1,2,4-triazin-1-oxid
3-Phenylsulfonyl-7-chlor-benzo-1,2,4-triazin-1-oxid
3-(4-Methyl-phenylsulfonyl)-7-chlor-benzo-1,2,4-triazin-1-oxid
3-(4-Chlor-phenylsulfonyl)-7-chlor-benzo-1,2,4-triazin-1-oxid
3-(4-Methoxy-phenylsulfonyl)-7-chlor-benzo-1,2,4-triazin-1-oxid
3-(4-Nitro-phenylsulfonyl)-7-chlor-benzo-1,2,4-triazin-1-oxid
3-Methylsulfonyl-5,7-dichlor-benzo-1,2,4-triazin-1-oxid
3-Phenylsulfonyl-5,7-dichlor-benzo-1,2,4-triazin-1-oxid
3-Methylsulfonyl-7-brom-benzo-1,2,4-triazin-1-oxid
3-Phenylsulfonyl-7-brom-benzo-1,2,4-triazin-1-oxid
3-Methylsulfonyl-7-trifluormethyl-benzo-1,2,4-triazin-1-oxid.

Die erfindungsgemäßen Verbindungen der Formel (I) weisen starke antimikrobielle, insbesondere antimykotische Wirkungen auf. Sie zeigen ein breites Wirkungsspektrum *in vitro*, das Dermatophyten, Hefen, Schimmelpilze und biphasische Pilze umfaßt. Besonders hervorzuheben ist eine rasch eintretende, gute sporozide Wirksamkeit gegen Pilzsporen.

Als Indikationsgebiete in der Humanmedizin können beispielsweise genannt werden :

Dermatomykosen und Systemmykosen, die durch Trichophyton mentagrophytes und andere Trichophyton-Arten, Mikrosporon-Arten, Epidermophyton floccosum, Sproßpilze und biphasische Pilze sowie Schimmelpilze hervorgerufen werden. Als Indikationsgebiet in der Tiermedizin können beispielsweise aufgeführt werden :

Alle Dermatomykosen und Systemmykosen, insbesondere solche, die durch die oben genannten Erreger hervorgerufen werden. Außerdem weisen die erfindungsgemäßen Wirkstoffe bei geringer Toxizität und guter Verträglichkeit eine starke antibakterielle Wirksamkeit auf.

Die genannten Eigenschaften ermöglichen die Verwendung der neuen Verbindungen als Wirkstoffe

außer in der Medizin auch im Materialschutz, insbesondere im Haushalts- und Gewerbesektor mit vorbeugender und Pilzbefall verhindernder Wirkung, sowie zur Konservierung von anorganischen und organischen Materialien aller Art.

Beispiele von Materialien, die durch Pilzwachstum nachteilig beeinflußt werden, sind beispielsweise Kalkanstriche, Dispersionsfarben, Tapeten, Fugen von Kachelwänden, Möbel, Leder, Kunstleder, Kunststoffe, Kautschuk, Badematten, Duschverhänge, Textilien, Teppichböden und Zeltausrüstungen.

Beispiele von insbesondere organischen Materialien, die zur Konservierung infrage kommen, sind beispielsweise Polymere, Schmiermittel, Farben, Fasern, Leder, Papier und Holz, sowie auch Lebensmittel, Kosmetika, wie Cremes und Salben, oder auch Wasser.

Die erfindungsgemäßen Wirkstoffe sind gegen ein sehr breites Spektrum von Mikroorganismen wirksam. Mit ihrer Hilfe können z. B. Gram-negative und Gram-positive Bakterien und bakterienähnliche Mikroorganismen bekämpft sowie dir durch diese Erreger hervorgerufenen Erkrankungen verhindert, gebessert und/oder geheilt werden.

Besonders wirksam sind die erfindungsgemäßen Wirkstoffe gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden. Beispielsweise können lokale und/oder systemische Erkrankungen behandelt und/oder verhindert werden, die durch die folgenden Erreger oder durch Mischungen der folgenden Erreger verursacht werden :

Micrococcaceae, wie Staphylokokken, z. B. Staphylococcus aureus, Staph. epidermidis ;

Lactobacteriaceae, wie Streptokokken, z. B. Streptococcus pyogenes, α- bzw. β-hämolysierende Streptokokken, nicht (γ)-hämolysierende Streptokokken, Str. viridans, Str. faecalis (Enterokokken) ;

Corynebacteriaceae, wie Corynebakterien, z. B. Corynebacterium diphtheriae, C. pyogenes, C. diphtheroides, C. acnes, C. parvum ;

Enterobacteriaceae, wie Escherichiae-Bakterien der Coli-Gruppe : Escherichia-Bakterien, z. B. Escherichia coli, Klebsiella-Bakterien, z. B. K. pneumoniae, Proteae-Bakterien der Proteus-Gruppe : Proteus, z. B. Proteus vulgaris, Pr. mirabilis, Pseudomonadaceae, wie Pseudomonas-Bakterien, z. B. Pseudomonas aeruginosa.

Die obige Aufzählung von Erregern ist lediglich beispielhaft und keineswegs beschränkend aufzufassen.

Als Krankheiten, die durch die erfindungsgemäßen Wirkstoffe verhindert, gebessert und/oder geheilt werden können, seien beispielsweise genannt :

Erkrankungen der Atmungswege und des Rachenraumes ;

Otitis ; Pharyngitis ; Pyelonephritis ; Cystitis ; sowie lokale Infektionen z. B. der Haut und lokal zugänglicher Schleimhäute.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Lösungen, Suspensionen und Emulsionen, Pasten, Salben, Gele, Cremes, Lotions, Puder und Sprays genannt.

Der oder die Wirkstoffe können gegebenenfalls mit einem oder mehreren der oben angegebenen Trägerstoffen auch in mikroverkapselter Form vorliegen.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Aethylacetat, Benzylalkohol, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z. B. Wasser, Aethylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Betonit, Agar-Agar und Tragant oder Gemische dieser Stoffe enthalten.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Im allgemeinen hat es sich sowohl in der Human- als auch in der Veterinärmedizin als vorteilhaft

erwiesen, den oder die erfindungsgemäßen Wirkstoffe in Gesamtmengen von etwa 10 bis 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Für die Anwendung im technischen Bereich bzw. auf dem Hygienesektor können die erfindungsgemäßen Wirkstoffe in nicht modifizierter Form oder mit einem Träger, z. B. dispersiert auf einem feinverteilten Feststoff, oder als Staub eingesetzt werden. Solche Gemische können auch in Wasser unter Zuhilfenahme eines Netzmittels dispersiert werden und die resultierenden Emulsionen können als Spray verwendet werden. Bei anderen Arbeitsweisen können die Produkte als Wirkstoffe in Lösungsmittel-Lösungen, Öl-in-Wasser- oder Wasser-in-Öl-Emulsionen verwendet werden. Die Mischungen können als Konzentrate formuliert werden und danach mit weiteren flüssigen oder festen Hilfsstoffen verdünnt werden, um die Endbehandlungsmischung herzustellen. Gute Ergebnisse werden insbesondere dann erhalten, wenn Mischungen verwendet werden, die 0,5 bis 3 % Wirkstoff enthalten.

## Beispiele

### Vorstufen A

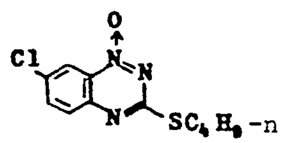

In die Mischung aus 9 g (0,1 Mol) n-Butylmercaptan, 9,1 g (0,1 Mol) konz. Natronlauge und 100 ml Dioxan werden bei Raumtemperatur unter schwachem Kühlen 21,6 g 3,7-Dichlor-benzo-1,2,4-triazin-1-oxid portionsweise eingetragen. Man rührt noch 1 Stde. bei Raumtemperatur und 2 Stdn. bei 50° nach und gießt dann das Reaktionsgemisch in Wasser ein. Die anfangs ölige Fällung kristallisiert beim längeren Kühlen. Die Kristalle werden abgesaugt und aus wenig Waschbenzin umkristallisiert. Man erhält 22,7 g (84 % d. Th.) 7-Chlor-3-butylmercapto-benzo-1,2,4-triazin-1-oxid vom Schmelzpunkt F : 58-60 °C.

In entsprechender Weise werden hergestellt :

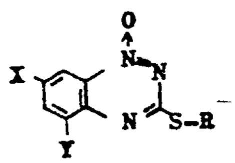

| X | Y | R | F(°C) | Umkrist. aus |
|---|---|---|---|---|
| Cl | H | $CH_3$ | 137-138 | Waschbenzin |
| Cl | H | $CH_2$- 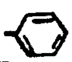 | 101-102 | Waschbenzin |
| Cl | H | 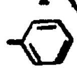 | 187-188 | Toluol |
| Cl | Cl | $CH_3$ | 145-146 | Tetra/Ligroin |
| Cl | Cl | n-$C_4H_9$ | 54-55 | Waschbenzin |
| Cl | Cl | 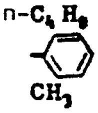 | 155-156 | Waschbenzin |
| Br | H | $CH_3$ | 142-143 | Waschbenzin |
| $F_3C$ | H | $CH_3$ | 111-112 | Waschbenzin |

### Vorstufen B

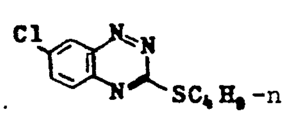

In einem VA-Rührautoklaven wird eine Lösung von 27,0 g (0,1 Mol) 7-Chlor-3-butylmercapto-benzo-1,2,4-triazin-1-oxid in 175 ml Dioxan mit 5 g Raney-Nickel vorgelegt. Bei 30 °C werden 15 atm. Wasserstoff aufgedrückt. Man rührt während 5 Stdn. bei 30-50° nach und drückt dabei weiteren Wasserstoff auf, bis der Druck konstant bleibt. Der Autoklav wird entspannt, der Katalysator abgesaugt und das Filtrat im Vakuum eingedampft. Der Rückstand wird aus Ligroin umkristallisiert. Man erhält 8 g (85 % d. Th.) 7-Chlor-3-n-butylmercapto-benzo-1,2,4-triazin vom Schmelzpunkt F : 66-68 °C.

In analoger Weise werden gewonnen :

| X | Y | R | F(°C) | umkrist. aus |
|---|---|---|---|---|
| Cl | H | $CH_3$ | 105-106 | Toluol |
| Cl | H | 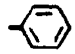 | 155-156 | Toluol |
| Cl | Cl | $CH_3$ | 146-148 | Tetra/Ligroin |
| Br | H | $CH_3$ | 108-110 | Waschbenzin |

Beispiel 1

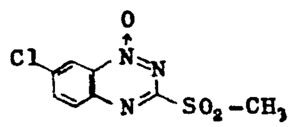

22,8 g (0,1 Mol) 7-Chlor-3-methylmercapto-benzo-1,2,4-triazin-1-oxid werden in einem Gemisch aus 200 ml Essigsäure und 60 ml Wasser suspendiert. Unter Einhaltung einer Temperatur von 0-5° leitet man Chlor bis zur Sättigung ein. Anschließend wird der Ueberschuß an Chlor durch Einleiten von Stickstoff entfernt. Das Reaktionsgemisch wird mit 1 ltr. Wasser verdünnt. Die abgeschiedenen Kristalle werden abgesaugt und aus wenig Toluol umkristallisiert. Man erhält 18,7 g (72 % d. Th.) 7-Chlor-3-methylsulfonyl-benzo-1,2,4-triazin-1-oxid vom Schmelzpunkt F : 213-214 °C.

In analoger Weise erhält man :

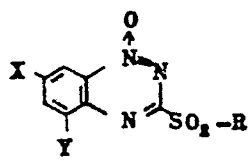

| Beispiel Nr. | X | Y | R | F(°C) | umkrist. aus |
|---|---|---|---|---|---|
| 2 | Cl | H | $n-C_4H_9$ | 160 | Butanol |
| 2a | Cl | H | $n-C_3H_7$ | 198-200 | Toluol |
| 3 | Cl | H | $CH_2$-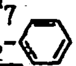 | 105-106 | Waschbenzin |
| 4 | Cl | H | -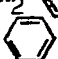 | 202-204 | Toluol |
| 5 | Cl | Cl | $CH_3$ | 168-170 | Essigester/Ligroin |
| 5a | Cl | H | -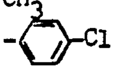-Cl | 210-212 | Waschbenzin |
| 5b | Cl | H | -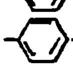-$CH_3$ | 248-250 | Toluol |
| 5c | Cl | H | -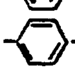-$C(CH_3)_3$ | 210-212 | Waschbenzin |
| 6 | Cl | Cl | $nC_4H_9$ | 113-114 | Äthanol |
| 7 | Cl | Cl | -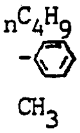 | 199-200 | Toluol |
| 8 | Br | H | $CH_3$ | 228-230 | Dioxan |
| 9 | $F_3C$ | H | $CH_3$ | 189-190 | Toluol |

Beispiel 10

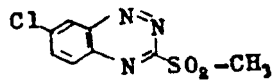

21,2 g (0,1 Mol) 7-Chlor-3-methylmercapto-benzo-1,2,4-triazin werden in einem Gemisch aus 300 ml Essigsäure und 120 ml Wasser suspendiert. Bei 0-5° wird gasförmiges Chlor bis zur Sättigung eingeleitet. Man rührt noch 2 Stdn. bei der gleichen Temperatur nach und entfernt dann den Ueberschuß an Chlor durch Einblasen von Stickstoff. Das Reaktionsgemisch wird mit 1 ltr. Wasser verdünnt, das kristalline Produkt abgesaugt. Nach Umkristallisieren aus Aethanol erhält man 19 g (73,5 % d. Th.) 7-Chlor-2-methylsulfonyl-benzo-1,2,4-triazin vom Schmelzpunkt F : 147-148 °C.

In entsprechender Weise gewinnt man :

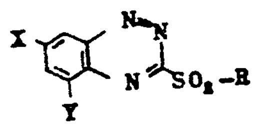

| Beispiel Nr. | X | Y | R | F(°C) | umkrist. aus |
|---|---|---|---|---|---|
| 11 | Cl | H | $n\text{-}C_4H_9$ | 90–92 | Waschbenzin |
| 12 | Cl | H | 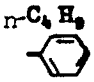 | 169–170 | Toluol |
| 13 | Cl | Cl | $CH_3$ | 137–138 | Toluol |
| 14 | Br | H | $CH_3$ | 167–168 | Essigester |

Beispiel A

Antimykotische In-vitro-Wirksamkeit

Versuchsbeschreibung :

Die minimalen Hemmkonzentrationen (MHK) gegen Pilze wurden im Reihenverdünnungstest mit Keiminokula von durchschnittlich 5 × $10^4$ Keimen/ml Substrat ermittelt. Als Nährmedium dienten

a) für Dermatophyten und Schimmelpilze : Sabouraud's milieu d'épreuve,

b) für Hefen : Fleischextrakt-Traubenzucker-Bouillon.

Die Bebrütungstemperatur betrug 28 °C, die Bebrütungsdauer lag bei 24 bis 96 Stunden.

Die Ergebnisse zeigen eine hohe antimykotische Wirkung.

Beispiel B

Antibakterielle In-vitro-Wirksamkeit

Versuchsbeschreibung :

Die minimalen Hemmkonzentrationen gegen Bakterien wurden im Agarverdünnungstest ermittelt. Dazu wurden verschiedene Präparatkonzentrationen zusammen mit dem Teststamm in flüssigem DST-Agar-Medium suspendiert und in Petrischalen (Durchmesser 5 cm) gegossen. Die Keimeinsaat pro Platte betrug 5 × $10^3$ Keime. Als MHK wird die geringste Präparatkonzentration bezeichnet, bei der innerhalb von 24 Std. keine Koloniebildung mehr stattfand.

Die Ergebnisse zeigen eine hohe antibakterielle Wirkung.

Beispiel C

Sporozide Wirksamkeit

Versuchsbeschreibung :

Es wurden Sporensuspensionen in physiologischer Kochsalzlösung hergestellt, die pro ml ca. $10^6$ Mikrokonidien von Trichophyton mentagrophytes enthielten. Nach Zugabe von 10, 50 und 100 mcg/ml

Wirkstoff wurden diese Sporensuspensionen bei 28 °C inkubiert, nach 24, 48, 96, 120 und 240 Stunden Proben entnommen, 1 : 1 000 verdünnt und dann auf Malzextrakt-Agarplatten homogen ausgespatelt. Nach 3 Tagen Inkubation bei 28 °C wurden die Pilzkolonien auf diesen Agarplatten ausgezählt als Maß für die Anzahl keimfähiger Sporen. Die Auswertung erfolgte im Vergleich zu Keimkontrollen ohne Wirkstoff.

Ergebnis :

Die Wirkstoffe gemäß den Herstellungsbeispielen 5, 11 und 14 zeigen bereits in Konzentrationen von 10 mcg/ml sehr gute sporozide Wirkung : nach 24 Stunden Einwirkungsdauer waren sämtliche eingesetzten Sporen abgetötet oder keimunfähig.

## Ansprüche

1. 3-Sulfonyl-benzo-1,2,4-triazine und -benzo-1,2,4-triazin-1-oxide der Formel (I)

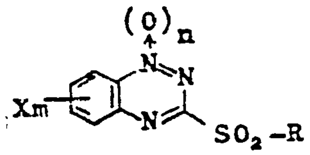

(I)

in der

X für Chlor, Brom oder Trifluormethyl,

R für $C_1$-$C_4$-Alkyl, einen gegebenenfalls durch Halogen, Nitro oder Methyl substituierten Benzylrest oder für einen gegebenenfalls durch Halogen, Nitro, Methyl oder Alkoxy mit 1 bis 4 C-Atomen substituierten Phenylrest,

m für 1 oder 2 und

n für 0 oder 1 stehen.

2. 7-Chlor-3-methylsulfonyl-benzo-1,2,4-triazin-1-oxid.
3. 7-Chlor-3-benzylsulfonyl-benzo-1,2,4-triazin-1-oxid.
4. 7-Chlor-3-phenylsulfonyl-benzo-1,2,4-triazin-1-oxid.
5. 5,7-Dichlor-3-phenylsulfonyl-benzo-1,2,4-triazin-1-oxid.
6. 7-Trifluormethyl-3-methylsulfonyl-benzo-1,2,4-triazin-1-oxid.
7. 7-Chlor-3-methylsulfonyl-benzo-1,2,4-triazin.
8. 7-Brom-3-methylsulfonyl-benzo-1,2,4-triazin.
9. Verfahren zur Herstellung von 3-Sulfonyl-benzo-1,2,4-triazinen und -benzo-1,2,4-triazin-1-oxiden der Formel (I)

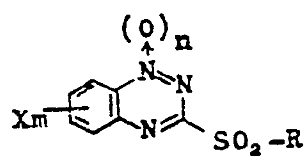

(I)

in der

X für Chlor, Brom oder Trifluormethyl,

R für $C_1$-$C_4$-Alkyl, einen gegebenenfalls durch Halogen, Nitro oder Methyl substituierten Benzylrest oder für einen gegebenenfalls durch Halogen, Nitro, Methyl oder Alkoxy mit 1 bis 4 C-Atomen substituierten Phenylrest,

m für 1 oder 2 und

n für 0 oder 1 stehen,

dadurch gekennzeichnet, daß man entweder 3-Sulfenyl-benzo-1,2,4-triazin oder entsprechende 1-Oxide der Formel (II)

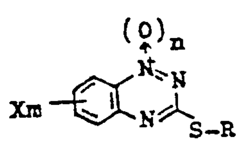

(II)

in der X, R, m und n die oben genannte Bedeutung besitzen, der Oxidation unterwirft, oder 3-Sulfonyl-benzo-1,2,4-triazin-1-oxide der Reduktion unterwirft.

10. Arzneimittel und antimikrobielle Mittel für den Materialschutz, gekennzeichnet durch einen Gehalt an mindestens einem der 3-Sulfonyl-benzo-1,2,4-triazine oder -benzo-1,2,4-triazin-1-oxide gemäß Anspruch 1.

**Claims**

1. 3-Sulphonyl-benzo-1,2,4-triazines and -benzo-1,2,4-triazine 1-oxides of the formula

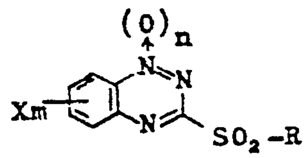

(I)

in which

X represents chlorine, bromine or trifluoromethyl,

R represents $C_1$-$C_4$-alkyl, a benzyl radical which is optionally substituted by halogen, nitro or methyl or a phenyl radical which is optionally substituted by halogen, nitro, methyl or alkoxy with 1 to 4 C atoms,

m represents 1 or 2 and

n represents 0 or 1.

2. 7-Chloro-3-methylsulphonyl-benzo-1,2,4-triazine 1-oxide.
3. 7-Chloro-3-benzylsulphonyl-benzo-1,2,4-triazine 1-oxide.
4. 7-Chloro-3-phenylsulphonyl-benzo-1,2,4-triazine 1-oxide.
5. 5,7-Dichloro-3-phenylsulphonyl-benzo-1,2,4-triazine 1-oxide.
6. 7-Trifluoromethyl-3-methylsulphonyl-benzo-1,2,4-triazine 1-oxide.
7. 7-Chloro-3-methylsulphonyl-benzo-1,2,4-triazine.
8. 7-Bromo-3-methylsulphonyl-benzo-1,2,4-triazine.
9. Process for the preparation of 3-sulphonyl-benzo-1,2,4-triazines and -benzo-1,2,4-triazine 1-oxides of the formula (I)

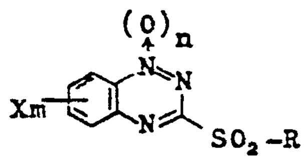

(I)

in which

X represents chlorine, bromine or trifluoromethyl,

R represents $C_1$-$C_4$-alkyl, a benzyl radical which is optionally substituted by halogen, nitro, or methyl or a phenyl radical which is optionally substituted by halogen, nitro, methyl or alkoxy with 1 to 4 C atoms,

m represents 1 or 2 and

n represents 0 or 1,

characterised in that either 3-sulphenyl-benzo-1,2,4-triazines or corresponding 1-oxides of the formula (II)

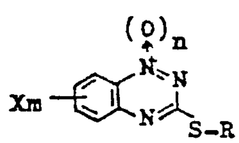

(II)

in which X, R, m and n have the abovementioned meaning, are subjected to oxidation of 3-sulphonyl-benzo-1,2,4-triazine 1-oxides are subjected to reduction.

10. Medicaments and antimicrobial agents for the protection of materials, characterised in that they contain at least one of the 3-sulphonyl-benzo-1,2,4-triazines or -benzo-1,2,4-triazine 1-oxides according to Claim 1.

**Revendications**

1. 3-sulfonyl-benzo-1,2,4-triazines et 1-oxydes de 3-sulfonyl-benzo-1,2,4-triazines, de formule

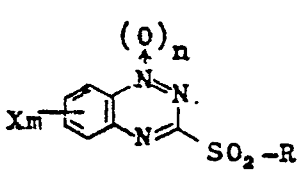

(I)

dans laquelle

X représente du chlore, du brome ou le groupe trifluorométhyle,

R est un groupe alkyle en $C_1$ à $C_4$, un reste benzyle éventuellement substitué par un halogène, un radical nitro ou méthyle ou un reste phényle éventuellement substitué par un halogène ou un radical nitro, méthyle ou alkoxy ayant 1 à 4 atomes de carbone,

m est égal à 1 ou 2 et

n est égal à 0 ou 1.

2. 1-oxyde de 7-chloro-3-méthylsulfonyl-benzo-1,2,4-triazine.

3. 1-oxyde de 7-chloro-3-benzylsulfonyl-benzo-1,2,4-triazine.

4. 1-oxyde de 7-chloro-3-phénylsulfonyl-benzo-1,2,4-triazine.

5. 1-oxyde de 5,7-dichloro-3-phénylsulfonyl-benzo-1,2,4-triazine.

6. 1-oxyde de 7-trifluorométhyl-3-méthylsulfonyl-benzo-1,2,4-triazine.

7. 7-chloro-3-méthylsulfonyl-benzo-1,2,4-triazine.

8. 7-bromo-3-méthylsulfonyl-benzo-1,2,4-triazine.

9. Procédé de production de 3-sulfonyl-benzo-1,2,4-triazines et de 1-oxydes de 3-sulfonyl-benzo-1,2,4-triazines de formule (I)

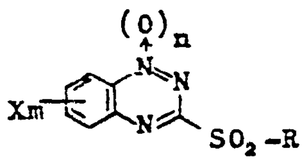

(I)

dans laquelle

X représente du chlore, du brome ou le groupe trifluorométhyle,

R est un groupe alkyle en $C_1$ à $C_4$, un reste benzyle éventuellement substitué par un halogène, un radical nitro ou méthyle ou un reste phényle éventuellement substitué par un halogène ou un radical nitro, méthyle ou alkoxy ayant 1 à 4 atomes de carbone,

m est égal à 1 ou 2 et

n est égal à 0 ou 1,

caractérisé en ce que ou bien on soumet à l'oxydation des 3-sulfényl-benzo-1,2,4-triazines ou les 1-oxydes correspondants de formule (II)

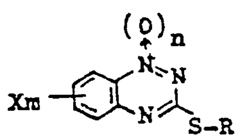

(II)

dans laquelle X, R, m et n ont la définition mentionnée ci-dessus, ou bien on soumet des 1-oxydes de 3-sulfonyl-benzo-1,2,4-triazines à la réduction.

10. Médicaments et compositions antimicrobiennes pour la production de matières, caractérisés par une teneur en au moins l'une des 3-sulfonyl-benzo-1,2,4-triazines ou l'un des 1-oxydes de 3-sulfonyl-benzo-1,2,4-triazines suivant la revendication 1.